Europäisches Patentamt

European Patent Office

Office européen des brevets

(11) Publication number: **0 379 619**
**A1**

## EUROPEAN PATENT APPLICATION

(21) Application number: **89101368.2**

(51) Int. Cl.5: **C12Q 1/40**

(22) Date of filing: **26.01.89**

(43) Date of publication of application:
**01.08.90 Bulletin 90/31**

(84) Designated Contracting States:
**DE FR GB IT**

(71) Applicant: **ORIENTAL YEAST CO., LTD.**
**10-gou, 6-ban, 3-chome, Azusawa Itabashi-ku**
**Tokyo 174(JP)**

(72) Inventor: **Takata, Shigeru**
**4-27, Minamiasahigaoka-cho**
**Tondabayashi-shi Osaka-fu(JP)**
Inventor: **Fujita, Tuyosi**
**4-3-17, Satsukigaoka**
**Ikeda-shi Osaka-fu(JP)**
Inventor: **Takagawara, Isamu**
**5-7-13, Yamatohigashi**
**Kawanishi-shi Hyogo-ken(JP)**
Inventor: **Marui, Yoji**
**3-4, Kosaka**
**Higashiosaka-shi Osaka-fu(JP)**
Inventor: **Hayashi, Chozo**
**1-2-610, Takahata-cho**
**Nishinomiya-shi Hyogo-ken(JP)**

(74) Representative: **Thomsen, Dieter, Dr.rer.nat.**
**Kaiser-Ludwig-Platz 6**
**D-8000 München 2(DE)**

(54) **Method of selective assay for alpha-amylase isozymes.**

(57) The present invention relates to a method for selective assay of $\alpha$-amylase isozyme using as a substrate a non-reducing-end and reducing-end modified oligosaccharide, specifically, 4-nitrophenyl O-(4,6-O-benzyliden)-$\alpha$-D-glucopyranosyl-$\{(1\rightarrow4)$-O-$\alpha$-D-glucopyranosyl$\}_5$-$(1\rightarrow4)$-$\alpha$-D-glucopyranoside (hereafter referred to as benzylidene-$G_7$PNP) rapidly and accurately.

EP 0 379 619 A1

# METHOD OF SELECTIVE ASSAY FOR α-AMYLASE ISOZYMES

Field of the Invention

The present invention relates to a novel method of selective assay for α-amylase isozymes.

More particularly, the present invention relates to a method for selective assay of α-amylase isozyme using as a substrate a non-reducing-end and reducing-end modified oligosaccharide, specifically, 4-nitrophenyl O-(4,6-O-benzyliden -α-D-glucopyranosyl-{(1→4)-O-α-D-glucopyranosyl}$_5$-(1→4)-α-D-glucopyranoside (hereafter referred to as benzylidene-G$_7$PNP) rapidly and accurately.

Accurate selective assay for α-amylase isozymes according to the present invention serves diagnosis for diseases of digestive tracts such as salivary gland, pancreas, etc. and is of great benefit to the medical field and the field of diagnostics.

Prior Art and Problems to be solved by the Invention

In general, α-amylase is a hydrolase which distributed in various living organisms and is secreted mainly from salivary gland and pancreas in human. It is known that its activity value is greatly varied depending upon diseases of salivary gland and pancreas and further diseases of liver, digestive tracts, etc.

It is also important for diagnosis of diseases of salivary gland, pancreas, liver, etc. to measure α-amylase activity by differentially determine α-amylase isozymes derived from salivary gland (S-amylase) and from pancreas (P-amylase), namely, S-amylase and P-amylase. Such a measurement has come to be applied to conventional clinical tests.

With respect to selective assay for α-amylase isozymes, many methods such as electrophoresis, gel filtration, an immunological method and the like have been hitherto reported.

However, these methods require complicated operations and long periods of time and hence, encounter many problems as a method for treating samples rapidly and accurately required in the field of clinical tests. Thus, the methods could hardly be said to be practical.

As a means for solving these problems, a method for selective assay of α-amylase isozymes using α-amylase inhibitor has been reported by O'Donnell et al. (J. Clin. Chem., 23, 560 (1977)).

The method comprises calculating S-amylase and P-amylase from the total amylase activity measured and the amylase activity when the inhibitor is added, utilizing a difference between S-amylase and P-amylase in inhibition by an α-amylase inhibitor. Its theory is clear and it was expected that the method could soon be practically used.

However, substrate used in this method was amylose so that a problem still remains on operability and reproducibility.

Means for solving the Problems

In order to make the selective assay for α-amylase isozymes using α-amylase inhibitors more accurate, the present inventors have made extensive investigations and as a result, have come to accomplish a method for rapid and more accurate selective assay of α-amylase isozymes using non-reducing-end and reducing-end modified oligosaccharides.

A specific example of substrate used in the present invention is benzylidene-G$_7$PNP. In benzylidene-G$_7$PNP, its non-reducing-end is modified by benzylidene group and its reducing-end is modified by 4-nitrophenyl group. It is assumed that α-amylase would act on benzylidene-G$_7$PNP and further by the action of glucoamylase and α-glucosidase, 4-nitrophenol would be released and accurately measured.

In the present invention, amylase activity is measured using an α-amylase inhibitor, wherein benzylidene-G$_7$PNP is used as a substrate and selective assay of α-amylase isozymes is performed.

That is, activity values of standard P-amylase and standard S-amylase as well as total amylase in a sample when no α-amylase inhibitor is added and activity values of these amylases when an α-amylase inhibitor is added are determined; and selective assay of α-amylase isozymes is calculated according to the following equations. If activities of standard S-amylase and standard P-amylase are decreased to x% and y%, respectively:

$$T\text{-Amy} = S\text{-Amy} + P\text{-Amy}$$

$$
\left.
\begin{aligned}
I\text{-Amy} &= \frac{x \cdot S\text{-Amy}}{100} + \frac{y \cdot P\text{-Amy}}{100} \\[2mm]
S\text{-Amy} &= \frac{y \cdot T\text{-Amy} - 100 \cdot I\text{-Amy}}{y - x} \\[2mm]
P\text{-Amy} &= T\text{-Amy} - S\text{-Amy}
\end{aligned}
\right\} \quad \text{(I)}
$$

and from activity values of T-Amy and I-Amy, activity values of S- and P-amylase can be determined. In the equations, abbreviations have the following meanings.

T-Amy : total amylase activity

P-Amy : pancreas-derived amylase activity

S-Amy : salivary gland-derived amylase activity

I-Amy : total amylase activity when an inhibitor is added

Next, examples of the present invention are shown below.

Example 1

After 120 µl of P-amylase or S-amylase having a known concentration was mixed with 20 µl of wheat-derived α-amylase inhibitor (1.4 µg/ml), the mixture was allowed to stand at 37°C for 5 minutes. 20 µl of the mixture was added to the following reagent (Reagent 1). Based on change in absorbance at 405 nm, an inhibitory rate of each amylase isozyme by α-amylase inhibitor was determined.

| (Reagent 1) | | | |
|---|---|---|---|
| | Benzylidene-G$_7$PNP | 1 | mM |
| | Glucoamylase | 20 | U/ml |
| | α-Glucosidase | 10 | U/ml |
| | α-Cyclodextrin | 10 | mM |
| | Sodium chloride | 20 | mM |
| | Calcium acetate | 2 | mM |
| | Triton X = 405 | 0.1 | % |
| | NaN$_3$ | 0.03 | % |
| | BSA | 0.01 | % |
| | PIPES | 0.1 | M (pH 7.0) |

Each inhibitory rate was as follows.

Inhibitory rate:

P-amylase = 30%

S-amylase = 90%

Next, similar procedures were performed using 20 µl of samples containing P-amylase and S-amylase having known concentrations. Further 0.1 M-PIPES (pH 7.0) was used instead of the α-amylase inhibitor to determine the total amylase activity. Based on equation [I], a concentration of each amylase isozyme was measured. The results are shown in Table 1 below.

Table 1

| Sample | P/S Ratio (known) | Measurement Value According to This Invention (U/l) | | | |
|---|---|---|---|---|---|
| | | Total Amylase | P-Amylase | S-Amylase | P/S Ratio |
| A | 1.23 | 48 | 26 | 22 | 1.18 |
| B | 2.00 | 53 | 36 | 17 | 2.12 |
| C | 0.59 | 83 | 29 | 54 | 0.54 |

Example 2

To 2.45 ml of the following reagent (Reagent 2):

| (Reagent 2) | | | |
|---|---|---|---|
| | Glucoamylase | 20.4 | U/ml |
| | α-Glucosidase | 10.2 | U/ml |
| | α-Cyclodextrin | 10.2 | mM |
| | Sodium chloride | 20.4 | mM |
| | Calcium acetate | 2.0 | mM |
| | Triton X = 405 | 0.1 | % |
| | $NaN_3$ | 0.03 | % |
| | BSA | 0.01 | % |
| | α-Amylase inhibitor | 1.4 | μg/ml |
| | PIPES | 0.1 | M (pH 7.0) |

was separately added 20 μl each of P-amylase or S-amylase having a known concentration. After the mixture was incubated at 37° C for 5 minutes, 50 μl of 50 mM benzylidene-$G_7$PNP solution was added to the mixture. Based on change in absorbance at 405 nm, an inhibitory rate of each amylase isozyme by α-amylase inhibitor was determined.

Each inhibitory rate was as follows.

Inhibitory rate:

P-amylase = 72%

S-amylase = 97%

Next, similar procedures were performed using 20 μl of samples containing P-amylase and S-amylase having known concentrations. Further the total amylase activity was determined using Reagent 2 added with no α-amylase inhibitor. Based on equation [I], a concentration of each amylase isozyme was measured.

The results are shown in Table 2 below.

Table 2

| Sample | P/S Ratio (known) | Measurement Value According to This Invention (U/l) | | | |
|---|---|---|---|---|---|
| | | Total Amylase | P-Amylase | S-Amylase | P/S Ratio |
| D | 0.77 | 162 | 68 | 94 | 0.72 |
| E | 0.22 | 124 | 18 | 106 | 0.17 |
| F | 1.02 | 70 | 36 | 34 | 1.06 |

Example 3

To 2.5 ml of the following reagent (Reagent 3):

| (Reagent 3) | | | |
|---|---|---|---|
| | Benzylidene-$G_7$PNP | 1 | mM |
| | Glucoamylase | 20 | U/ml |
| | α-Glucosidase | 10 | U/ml |
| | α-Cyclodextrin | 10 | mM |
| | Sodium chloride | 20 | mM |
| | Calcium acetate | 2 | mM |
| | Triton X = 405 | 0.1 | % |
| | NaN$_3$ | 0.03 | % |
| | BSA | 0.01 | % |
| | α-Amylase inhibitor | 1.4 | μg/ml |
| | PIPES | 0.1 | M (pH 7.0) |

was separately added 20 μl each of P-amylase or S-amylase having a known concentration. Based on change in absorbance at 405 nm, an inhibitory rate against each amylase isozyme was determined.

Each inhibitory rate was as follows.

Inhibitory rate:

P-amylase = 55%

S-amylase = 90%

Next, similar procedures were performed using 20 μl of samples containing P-amylase and S-amylase having known concentrations. Further the total amylase activity was determined using Reagent 3 added with no α-amylase inhibitor. Based on equation [I], a concentration of each amylase isozyme was measured.

The results are shown in Table 3 below.

Table 3

| Sample | P/S Ratio (known) | Measurment Value According to This Inventin (U/l) | | | |
|---|---|---|---|---|---|
| | | Total Amylase | P-Amylase | S-Amylase | P/S Ratio |
| G | 0.59 | 93 | 33 | 60 | 0.55 |
| H | 1.11 | 38 | 19 | 19 | 1.00 |
| I | 0.93 | 101 | 50 | 51 | 0.98 |

**Claims**

1. A method for selective assay of α-amylase isozyme characterized by using a non-reducing-end and reducing-end modified oligosaccharide as a substrate.

2 A method for selective assay of α-amylase isozyme as claimed in claim 1, wherein said substrate is 4-nitrophenyl O-(4,6-O-benzylidene)-α-D-glucopyranosyl-{(1→4)-O-α-D-glucopyranosyl}$_5$-(1→4)-α-D-glucopyranoside.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | CLINICAL CHEMISTRY, vol. 33, no. 4, April 1987, pages 524-528, Washington, DC, US; G. DUPUY et al.: "Rapid determination of alpha-amylase activity by use of a new chromogenic substrate" * Whole article * | 1,2 | C 12 Q   1/40 |
| A | CLINICAL CHEMISTRY, vol. 32, no. 8, August 1986, pages 1577-1580, Washington, DC, US; A. JIMENEZ et al.: "Catalytic concentrations of amylase isoenzymes: an assay with wheat-germ inhibitor and 4-nitrophenylmaltopentaoside plus 4-nitrophenylmaltohexaoside as substrate" * Whole article * | 1,2 | |
| A | US-A-4 337 309  (McGEENEY) * Whole document * | 1,2 | |
| Y | EP-A-0 252 525  (WAKO PURE CHEMICAL INDUSTRIES LTD) * Abstract; page 5, example V; page 6, table 1; claims 1,2,5,8 * | 1,2 | **TECHNICAL FIELDS SEARCHED (Int. Cl.5)**<br><br>C 12 Q |
| Y | EP-A-0 260 414  (WAKO PURE CHEMICAL INDUSTRIES LTD) * Abstract; page 5, line 36 * | 1,2 | |
| Y | EP-A-0 171 960  (GENZYME CORP.) * Whole document * | 1,2 | |
| Y | DE-A-3 738 477  (OGAWA ZENSUKE, FUJISAWA, KANAGAWA, JP) * Whole document *                      -/- | | |

The present search report has been drawn up for all claims

| Place of search | Date of completion of the search | Examiner |
|---|---|---|
| THE HAGUE | 26-09-1989 | OSBORNE H.H. |

EPO FORM 1503 03.82 (P0401)

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 104 047 (WAKO PURE CHEMICAL INDUSTRIES LTD) * Abstract; pages 4-6, lines 9-11; pages 24-28 * --- | 1 | |
| Y | EP-A-0 104 047 ----- | 2 | |

TECHNICAL FIELDS SEARCHED (Int. Cl.5)

The present search report has been drawn up for all claims

| Place of search | Date of completion of the search | Examiner |
|---|---|---|
| THE HAGUE | 26-09-1989 | OSBORNE H.H. |